# EUROPEAN PATENT SPECIFICATION

(11) **EP 4 154 970 B1**
(45) Date of publication and mention of the grant of the patent: **08.04.2026**
(21) Application number: 21809241.9
(22) Date of filing: 11.05.2021
(51) Int. Cl.: C08F 290/06, A61Q 1/00, A61Q 19/00, C08F 293/00, A61Q 5/00, A61K 8/06, A61K 8/891, B01J 13/00

(54) **USE OF A DIBLOCK COPOLYMER AS EMULSIFIER**
VERWENDUNG EINES DIBLOCKCOPOLYMERS ALS EMULGATOR
UTILISATION D'UN COPOLYMÈRE DIBLOC COMME ÉMULSIFIANT

(30) Priority: 22.05.2020 JP 2020089623
(43) Date of publication of application: 29.03.2023
(73) Proprietor: Shin-Etsu Chemical Co., Ltd., Tokyo 100-0005 (JP)
(72) Inventor: IMAI, Taro, Annaka-shi, Gunma 379-0224 (JP)
(74) Representative: Angerhausen, Christoph
(86) International application number: PCT/JP2021/017868
(87) International publication number: WO 2021/235274

(56) References cited:
- EP-A1- 3 527 592
- EP-A2- 0 452 098
- JP-A- 2017 218 595
- JP-A- 2019 137 775
- JP-A- H11 114 401
- ALLEN H. GABOR ET AL: "Group-Transfer Polymerization of tert -Butyl Methacrylate and [3-(Methacryloxy)propyl]pentamethyldisiloxane:? Synthesis and Characterization of Homopolymers and Random and Block Copolymers", CHEMISTRY OF MATERIALS, vol. 8, no. 9, 1 January 1996 (1996-01-01), US, pages 2272 - 2281, XP055600785, ISSN: 0897-4756, DOI: 10.1021/cm960012c
- GABOR, A. H. ET AL.: "Group-Transfer Polymerization of tert-Butyl Methacrylate and [3- (Methacryloxy)propyl] pentamethyldisiloxane: Synthesis and Characterization of Homopolymers and Random and Block Copolymers", CHEM. MATER., vol. 8, 1996, pages 2272 - 2281, XP055600785, DOI: 10.1021/cm960012c

## Description

### TECHNICAL FIELD

The present invention relates to an emulsifier exhibiting a high preservation stability and having an excellent emulsifying capability in a wide range of emulsification compositions, particularly to a (meth)acryl silicone-based diblock copolymer.

### BACKGROUND ART

Generally, in the case of a water-in-oil type emulsion composition, in order to obtain an emulsion composition that is smooth, has a little stickiness and is superior in water repellency, silicone oils are often used as oil agents; however, as for a water-in-oil type emulsion composition containing such silicone oil, it has been difficult to obtain an emulsion superior in stability even when using an emulsifier such as a polyoxyalkylene fatty acid ester-based emulsifier that has been conventionally used. Here, with regard to an emulsion whose oil phase is a silicone oil, there are widely known methods in which used as a surfactant (emulsifier) is a polyoxyalkylene-modified organopolysiloxane (polyether-modified silicone) having a favorable compatibility to a silicone oil (e.g. Patent documents 1 to 5). Patent document 6 discloses methacrylic silicone graft copolymers for use as film former, thickener or dispersant.

However, even when using a polyether-modified silicone, if adding a large amount of a water phase component to achieve a wateriness, the stability of an emulsion may be impaired whereby the oil phase and water phase may be separated over time, or the emulsion may not be able to be obtained in the first place. Here, in general, there is now considered a method for achieving a stability of an emulsion by either increasing the viscosity of the oil phase or turning it into a gel.

However, a large amount of a thickener or a gelator will actually lead to a loss of wateriness, and thereby contribute to a sticky texture. Further, there has been a downside where a poor extensibility is observed when applied to the skin, which leads to an insufficient smooth texture. Thus, desired is an emulsifier allowing there to be stably contained a large amount of water without adding a thickener or a gelator.

### PRIOR ART DOCUMENTS

### Patent documents

Patent document 1: JP-A-Sho 61-293903
Patent document 2: JP-A-Sho 61-293904
Patent document 3: JP-A-Sho 62-187406
Patent document 4: JP-A-Sho 62-215510
Patent document 5: JP-A-Sho 62-216635
Patent document 6: EP 3527592 A1

### SUMMARY OF THE INVENTION

Problems to be solved by the invention

Thus, it is an object of the present invention to provide an emulsifier exhibiting a high preservation stability and having an excellent emulsifying capability in a wide range of emulsification compositions, particularly an emulsifier suitable for producing a water-in-oil type emulsion composition capable of containing a large amount of a water phase component. Further, it is also an object of the present invention to provide an emulsion composition using such emulsifier.

### Means to solve the problems

The inventor of the present invention diligently conducted a series of studies to achieve the above object, and completed the invention as follows. That is, the inventors found that the aforementioned problems can be solved by a diblock copolymer having, as its structural components, a hydrophobic silicone graft copolymer block, and a polar copolymer block having a particular polar group(s) serving as a functional group.

Thus, the present invention is to provide a use of a diblock copolymer as an emulsifier.

The invention relates to the use of a diblock copolymer as an emulsifier, wherein a main chain of the diblock copolymer is comprised of a silicone graft copolymer block represented by a formula [I] and a polar copolymer block represented by a formula [II], wherein one end structure of the main chain is represented by a formula [III], and the other end structure of the main chain is represented by a formula [IV], the formula [I] being expressed as 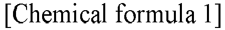 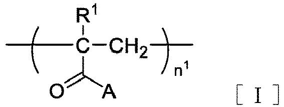
wherein R¹ represents a hydrogen atom or a methyl group,
   A represents an organopolysiloxane-containing group represented by a general formula (1) or an organopolysiloxane-containing group represented by a general formula (2),
   n¹ represents a number of repeating units and satisfies 1≤ n¹ ≤50,
wherein the general formula (1) is expressed as 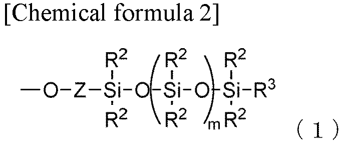
wherein the organopolysiloxane-containing group represented by the general formula (1) has a linear organopolysiloxane structure where a repeating unit number m is 0 to 100,
   Z represents a divalent organic group,
   each R² independently represents a saturated hydrocarbon group having 1 to 10 carbon atoms or a phenyl group,
   R³ represents a saturated hydrocarbon group having 1 to 10 carbon atoms,
   m is a number of 0 to 100, and
wherein the general formula (2) is expressed as
   [Chemical formula 3]

   -O-Lⁱ (2)
wherein the organopolysiloxane-containing group represented by the general formula (2) has a dendritic organopolysiloxane structure whose hierarchical number c is 1 to 10,
   Lⁱ is a silylorgano group represented by a general formula (3),
   i represents a number of each hierarchy of the dendritic structure and is each integer from 1 to c in both the general formulae (2) and (3), and is 1 in the general formula (2),
wherein the general formula (3) is expressed as 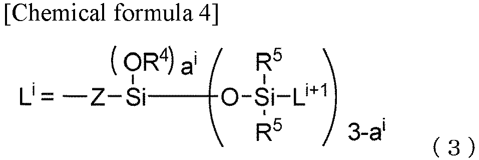
wherein Z represents a divalent organic group,
   R⁴ represents a saturated hydrocarbon group having 1 to 10 carbon atoms or a phenyl group,
   each R⁵ independently represents an alkyl group having 1 to 8 carbon atoms or a phenyl group,
   Lⁱ⁺¹ is the silylorgano group Lⁱ represented by the general formula (3) when the hierarchy i is lower than c (lower than the uppermost hierarchy); and is a hydrogen atom, a saturated hydrocarbon group having 1 to 10 carbon atoms or a phenyl group, when the hierarchy i is i=c (the uppermost hierarchy),
   aⁱ represents the number of OR⁴ groups in the hierarchy i and is a number of 0 to 3; the formula [II] being expressed as 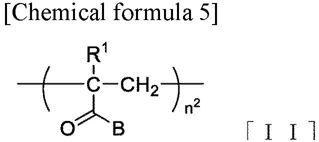
wherein R¹ represents a hydrogen atom or a methyl group,
   B is any group represented by a general formula (8') or (9'),
   n² represents a number of repeating units and satisfies 1≤ n²≤50; the formula [III] being expressed as 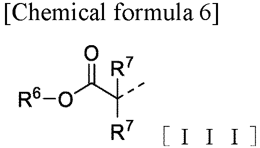
wherein R⁶ represents an alkyl group having 1 to 4 carbon atoms,
   each R⁷ independently represents a hydrogen atom or an alkyl group having 1 to 4 carbon atoms; the formula [IV] being expressed as 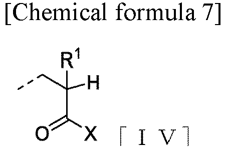
wherein R¹ represents a hydrogen atom or a methyl group,
   X represents the group represented by A in the formula [I] or the group represented by B in the formula [II].

### Effects of the invention

The emulsifier comprised of the diblock copolymer employed in the present invention has, as its structural components, a hydrophobic silicone graft copolymer block (segment [I]), and a polar copolymer block having a particular polar group(s) serving as a functional group (segment [II]). When preparing a water-in-oil type emulsion composition using such diblock copolymer, the interaction between the polar group(s) and a water phase and the interaction between the silicone moiety and an oil agent will turn into a stronger and more efficient interaction derived from the block structure, thereby allowing there to be obtained an emulsion composition having an excellent temporal stability in a wide range of emulsification compositions.

### MODE FOR CARRYING OUT THE INVENTION

The present invention is described in detail hereunder. Further, the term "(meth)acryl " used in this specification refers to methacryl and acryl. Similarly, the term "(meth)acrylate" used in this specification refers to methacrylic acid ester and acrylic acid ester.

### [Emulsifier]

A diblock copolymer employed in the present invention as an emulsifier has, in its main chain, a silicone graft copolymer block unit represented by the following formula [I] (segment [I]) and a polar copolymer block unit represented by the following formula [II] (segment [II]), where one end structure of the main chain is a structure represented by the following formula [III], and the other end structure of the main chain is a structure represented by the following formula [IV].

### Segment [I]

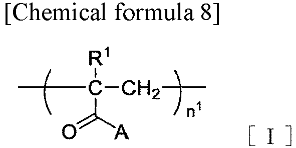

In the formula [I], R¹ represents a hydrogen atom or a methyl group. A represents an organopolysiloxane-containing group represented by the following general formula (1), or an organopolysiloxane-containing group represented by the following general formula (2). n¹ represents a number of the repeating units; n¹ is 1 to 50, preferably 1 to 20, more preferably 3 to 10.

The organopolysiloxane-containing group represented by the general formula (1) is a group having a linear organopolysiloxane structure where a repeating unit number m of a diorganosiloxy group is 0 to 100.

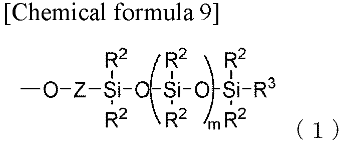

In the general formula (1), Z represents a divalent organic group, preferably a saturated hydrocarbon group having 2 to 12 carbon atoms, more preferably a propylene group. Each R² independently represents a saturated hydrocarbon group having 1 to 10 carbon atoms or a phenyl group, preferably a saturated hydrocarbon group having 1 to 5 carbon atoms, more preferably a methyl group. R³ represents a saturated hydrocarbon group having 1 to 10 carbon atoms, preferably a saturated hydrocarbon group having 1 to 5 carbon atoms, more preferably a methyl group. m is a number of 0 to 100, preferably a number of 1 to 60, more preferably a number of 5 to 30.

The organopolysiloxane-containing group represented by the general formula (2) is a group having a dendritically branched structure(s), where the number of such branched structures (hierarchical number c) is an integer of 1 to 10, preferably an integer of 1 to 6, more preferably an integer of 1 to 4.
[Chemical formula 10]

-O-Lⁱ (2)

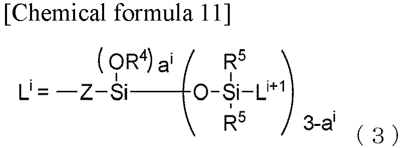

In the general formulae (2) and (3), i represents a number of each hierarchy of the dendritic structure, and is each integer from 1 to c.

In the general formula (2), Lⁱ is a silylorgano group represented by the general formula (3), and a hierarchy i in the general formula (2) is 1.

In the general formula (3), Z represents a divalent organic group, preferably a saturated hydrocarbon group having 2 to 12 carbon atoms, more preferably a propylene group. R⁴ represents a saturated hydrocarbon group having 1 to 10 carbon atoms or a phenyl group, preferably a saturated hydrocarbon group having 1 to 5 carbon atoms, more preferably a methyl group. Each R⁵ independently represents an alkyl group having 1 to 8 carbon atoms or a phenyl group, preferably an alkyl group having 1 to 3 carbon atoms, more preferably a methyl group. Lⁱ⁺¹ is the silylorgano group Lⁱ represented by the general formula (3) when the hierarchy i is lower than c (lower than the uppermost hierarchy); and is a hydrogen atom, a saturated hydrocarbon group having 1 to 10 carbon atoms or a phenyl group, when the hierarchy i is i=c (the uppermost hierarchy). As Lⁱ when i=c (the uppermost hierarchy), preferred is a saturated hydrocarbon group having 1 to 8 carbon atoms, more preferred is a saturated hydrocarbon group having 1 to 4 carbon atoms. aⁱ represents the number of the OR⁴ groups in the hierarchy i, and is a number of 0 to 3.

A dendritic organopolysiloxane of a hierarchical number 1 (c=1) is expressed by the following general formula (3-1) wherein L² represents a hydrogen atom, a saturated hydrocarbon group having 1 to 10 carbon atoms or a phenyl group.

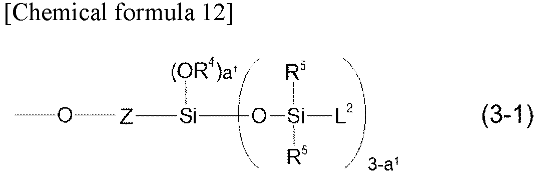

A dendritic organopolysiloxane of a hierarchical number 2 (c=2) is expressed by the following general formula (3-2) wherein L³ represents a hydrogen atom, a saturated hydrocarbon group having 1 to 10 carbon atoms or a phenyl group.

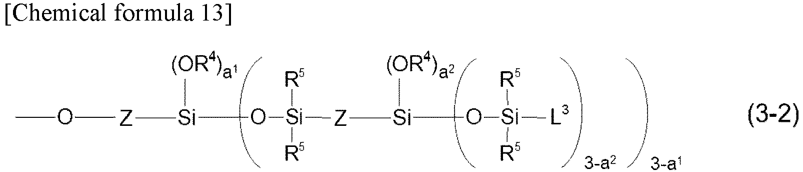

A dendritic organopolysiloxane of a hierarchical number 3 (c=3) is expressed by the following general formula (3-3) wherein L⁴ represents a hydrogen atom, a saturated hydrocarbon group having 1 to 10 carbon atoms or a phenyl group.

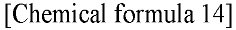

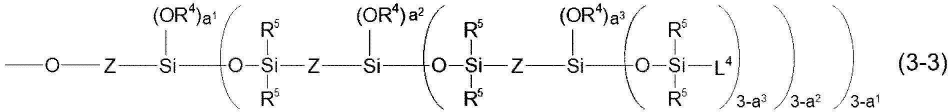

In the general formulae (3-1) to (3-3), Z, R⁴ and R⁵ are each identical to those described above, and each of a¹, a² and a³ is a number of 0 to 3.

### Segment [II]

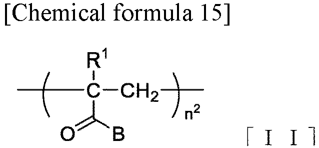

In the formula [II], R¹ represents a hydrogen atom or a methyl group. B is any group represented by a general formula (8') or (9'). Further, n² represents a number of the repeating units; n² is 1 to 50, preferably 1 to 20, more preferably 3 to 10.

[Chemical formulae 16] -O-CH₂-CH₂-N (R⁷) ₂ (8' ) -O-(C₂H₄O)_{n³}-R³ (9' )

(In the formula (8'), each R⁷ independently represents a hydrogen atom or an alkyl group having 1 to 4 carbon atoms. In the formula (9'), R³ represents a saturated hydrocarbon group having 1 to 10 carbon atoms. n³ represents a number of the repeating units, provided that 1≤n³≤10.)

### End structure [III]

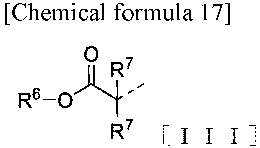

In the formula [III], R⁶ represents an alkyl group having 1 to 4 carbon atoms, preferably a methyl group. Each R⁷ independently represents a hydrogen atom or an alkyl group having 1 to 4 carbon atoms, preferably a methyl group.

### End structure [IV]

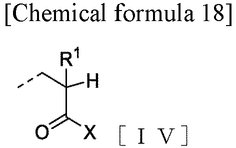

In the formula [IV], R¹ represents a hydrogen atom or a methyl group. X represents the group represented by A in the formula [I] or the group represented by B in the formula [II].

Each of the segments [I] and [II] may be composed of at least one kind of unit expressed by the formula [I] or at least one kind of unit expressed by the formula [II], or be composed of multiple kinds of units expressed by the formula [1] or multiple kinds of units expressed by the formula [II]. Further, the segments [I] and [II] that are sandwiched between the end structures [III] and [IV] are in a random order.

In the present invention, a diblock copolymer refers to a copolymer linking the segment [I] and the segment [II] as two segments having different physical properties such as polarity, water solubility, and presence or absence of affinity for powder. That is, the diblock copolymer of the present invention is a copolymer linking together the segments [I] and [II] of which the segment [I] is composed of consecutive units expressed by the formula [I], and the segment [II] is composed of consecutive units expressed by the formula [II]. If the segment [I] is composed of multiple kinds of units expressed by the formula [I], the segment [I] may have a block structure in which units expressed by an identical formula [I] are present in a consecutive manner, or a random structure in which units expressed by different formulae [I] are arranged randomly. Similarly, if the segment [II] is composed of multiple kinds of units expressed by the formula [II], the segment [II] may have a block structure in which units expressed by an identical formula [II] are present in a consecutive manner, or a random structure in which units expressed by different formulae [II] are arranged randomly.

In an embodiment, the diblock copolymer employed in the present invention as an emulsifier has a number average molecular weight (Mn) of 2,000 to 25,000, preferably 2,000 to 20,000, more preferably 3,000 to 15,000. Further in an embodiment, the polydispersity (Mw/Mn) thereof is 1.00 to 3.00, preferably 1.00 to 2.00, more preferably 1.05 to 1.60.

Further, the repeating unit number of each of the segments [I] and [II] is 1 to 50, preferably 1 to 20, more preferably 3 to 10.

A ratio between the repeating unit number of the segment [I] and the repeating unit number of the segment [II] i.e. a ratio of n²/n¹ which is a ratio between a polymerization degree n¹ of the segment [I] and a polymerization degree n² of the segment [II] is preferably 0.02 to 10, more preferably 0.05 to 5.

In the present invention, the molecular weight is a number average molecular weight measured by gel permeation chromatography (GPC) under the following conditions, using polystyrene as a reference substance.

### [Measurement conditions]

Measuring instrument: HLC-8320GPC (by Tosoh Corporation)
Developing solvent: Tetrahydrofuran (THF)
Flow rate: 0.600 mL/min
Detector: Refractive index detector (RI)
Column: TSK Guardcolumn Super H-H
   (4.6 mm I.D. × 35 mm)
   TSK gel Super H2500
   (Filler particle size: 3.0 µm, 6 mm I.D. × 150 mm)
   TSK gel Super HM-N
   (Filler particle size: 3.0 µm, 6 mm I.D. × 150 mm)
   (All manufactured by Tosoh Corporation)
Column temperature: 40°C
Sample injection volume: 50 µL (THF solution with a concentration of 0.3% by mass)

The diblock copolymer employed in the present invention as an emulsifier can be produced by a method having a step of performing group transfer polymerization on a monomer represented by a general formula (5), and a step of performing group transfer polymerization on a polar monomer represented by a general formula (6), using a compound represented by the following general formula (4) as an initiator. That is, the diblock copolymer employed in the present invention can be synthesized by sequentially performing group transfer polymerization on the monomer represented by the general formula (5) and on the polar monomer represented by the general formula (6), using the compound represented by the general formula (4) as an initiator; the monomer represented by the general formula (5) and the polar monomer represented by the general formula (6) can be subjected to group transfer polymerization in any order.

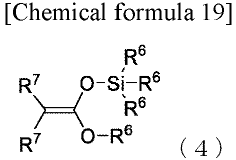

In the general formula (4), each R⁶ independently represents an alkyl group having 1 to 4 carbon atoms; each R⁷ independently represents a hydrogen atom or an alkyl group having 1 to 4 carbon atoms.

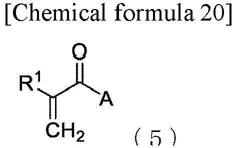

In the general formula (5), R¹ and A are defined as above in the formula [I].

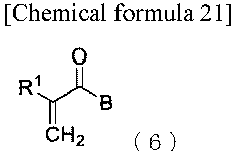

In the general formula (6), R¹ and B are defined as above in the formula [II].

As the initiator represented by the general formula (4), the following compounds may for example be used. The initiator usable in the production method of the diblock copolymer employed in the present invention shall not be limited to the initiators exemplified below.

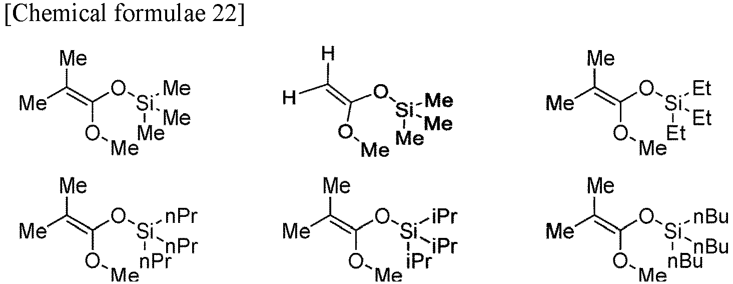

In these formulae, Me represents a methyl group, Et represents an ethyl group, nPr represents a n-propyl group, iPr represents an isopropyl group, and nBu represents a n-butyl group.

As the monomer represented by the general formula (5), there may be used for example the following monomers. The monomer usable in the production method of the diblock copolymer employed in the present invention shall not be limited to the monomers exemplified below.

When A in the general formula (5) is the organopolysiloxane-containing group represented by the general formula (1)

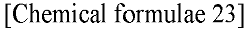

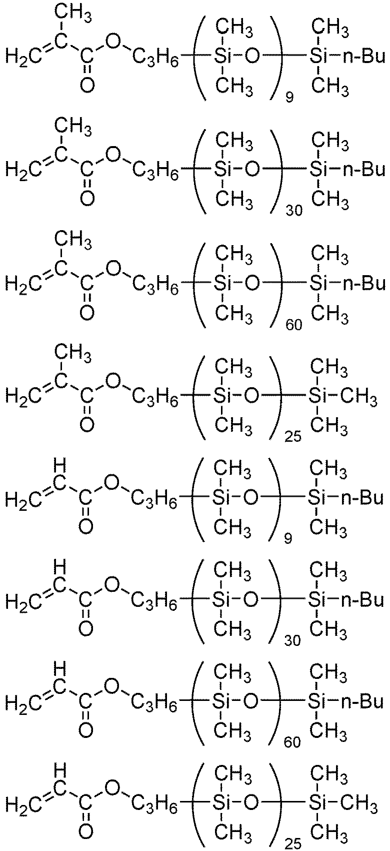

When A in the general formula (5) is the organopolysiloxane-containing group represented by the general formula (2)

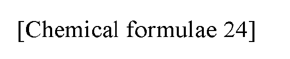

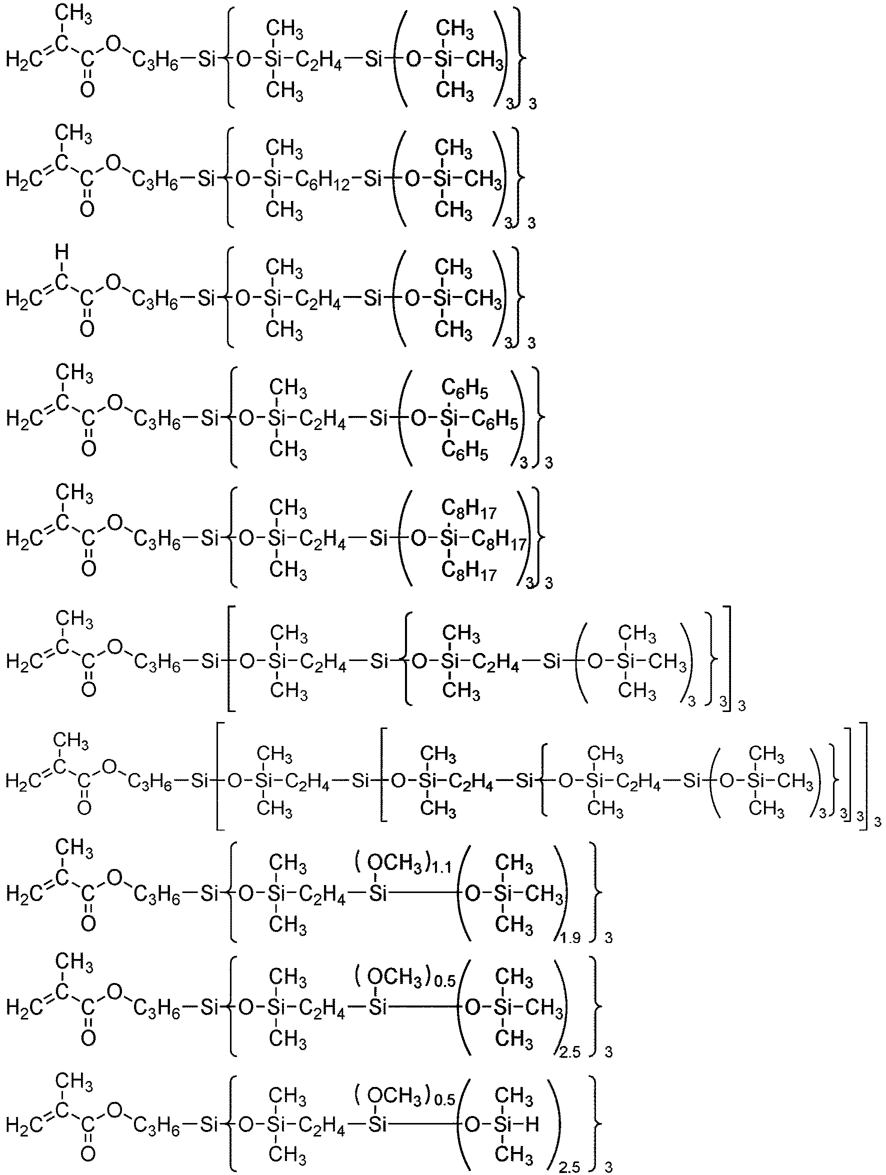

As the polar monomer represented by the general formula (6), there may be used for example the following polar monomers. The polar monomer usable in the production method employed in the diblock copolymer of the present invention shall not be limited to the polar monomers exemplified below.

Specifically, there may be listed, for example, an oxyalkylene-substituted (meth)acrylate such as tetrahydrofurfuryl (meth)acrylate, di(ethyleneglycol)monomethylether (meth)acrylate, furfuryl (meth)acrylate, 2-methoxyethyl (meth)acrylate, 2-butoxyethyl (meth)acrylate, 2-ethoxyethyl (meth)acrylate, 2-(methoxyethoxy)ethyl (meth)acrylate, allyloxyethyl (meth)acrylate, 1-ethoxybutyl (meth)acrylate, tetrahydro-4H-pyranyl-2 (meth)acrylate, ethyltriglycol (meth)acrylate, butyldiglycol (meth)acrylate, poly(propyleneglycol)dimethylether (meth)acrylate and poly(ethyleneglycol)alkylether (meth)acrylate; an aminoalkyl (meth)acrylate such as dimethylaminoethyl methacrylate and diethylaminoethyl methacrylate; and a (meth)acrylamide such as (meth)acrylamide, 4-(meth)acryloylmorpholine, N-tert-butyl (meth)acrylamide, N,N-dimethyl (meth)acrylamide, N,N-diethyl (meth)acrylamide, N-(butoxymethyl) (meth)acrylamide, N-[3-(dimethylamino)propyl] (meth)acrylamide, N-dodecyl (meth)acrylamide and N-isopropyl (meth)acrylamide.

As the polar monomer represented by the general formula (6), preferred is a polar monomer represented by the following general formula (8) or (9). 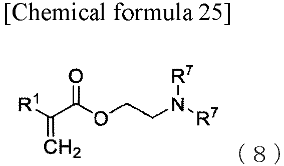 In the general formula (8), R¹ represents a hydrogen atom or a methyl group, preferably a methyl group. Each R⁷ independently represents a hydrogen atom or an alkyl group having 1 to 4 carbon atoms, preferably a methyl group and an ethyl group. 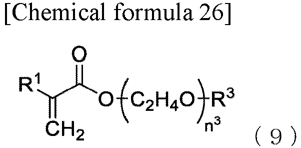 In the general formula (9), R¹ represents a hydrogen atom or a methyl group, preferably a methyl group. R³ represents a saturated hydrocarbon group having 1 to 10 carbon atoms, preferably a saturated hydrocarbon group having 1 to 5 carbon atoms, more preferably a methyl group. n³ represents a number of the repeating units, provided that 1≤n³≤10, preferably 2≤n³≤8.

As the polar monomer represented by the general formula (6), particularly preferred is a 2-(dimethylamino)ethyl methacrylate represented by a formula (8-1).

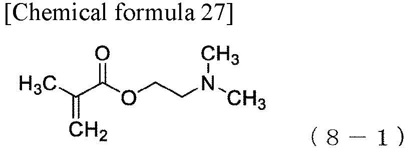

In the production method of the diblock copolymer employed in the present invention, group transfer polymerization is performed via the following two stages. In a first stage, any one of the monomer represented by the general formula (5) and the polar monomer represented by the general formula (6) is to be polymerized (the monomer to be polymerized in the first stage is referred to as a first monomer hereunder); subsequently, in a second stage, any one of the monomer represented by the general formula (5) and the polar monomer represented by the general formula (6), that was not polymerized in the first stage, is to be polymerized (the monomer to be polymerized in the second stage is referred to as a second monomer hereunder).

In the first stage, of the three components which are the compound represented by the general formula (4) and serving as an initiator, a catalyst, and the first monomer, two components are to be mixed together in advance, followed by adding and mixing the remaining one component thereinto so as to allow the polymerization of the first monomer to start taking place at first.

Next, after confirming that the polymerization reaction of the first monomer has stopped, the second monomer will be added to the reaction system so as to allow the polymerization of the second monomer to start taking place.

After confirming that the polymerization reaction of the second monomer has stopped, a reaction terminator will be added so as to end the reaction.

After the reaction was over, the diblock copolymer as the target product can be obtained by performing purification in a conventional manner where, for example, a solvent and the unreacted monomer are to be distilled away under a reduced pressure.

In the group transfer polymerization reactions of the first and second stages, it is preferred that a solvent be used.

As an example of a more specific production method, there may be employed the following method.

A catalyst is put into a thoroughly dried triple-necked flask, followed by adding a solvent thereto. Moreover, after adding and mixing the initiator represented by the general formula (4) thereinto, a dropping funnel is then used to deliver the first monomer by drops while performing stirring. The reaction solution is cooled according to the extent of heat generation so that the reaction solution will be maintained at an appropriate temperature. After the first monomer was delivered by drops, stirring will be performed continuously until the first monomer has been consumed, where the termination of the polymerization reaction of the first monomer will then be confirmed by confirming, via gel permeation chromatography (GPC) analysis or the like, an increase in molecular weight according to a preparation ratio between the initiator and the first monomer. Next, the second monomer will be delivered into this reaction system by drops while performing stirring. The reaction solution is cooled according to the extent of heat generation so that the reaction solution will be maintained at an appropriate temperature. After the second monomer was delivered by drops, stirring will be performed continuously until the second monomer dropped has been consumed, where a reaction terminator will be added in the end so as to end the reaction. After the reaction was over, the diblock copolymer as the target product can be obtained by performing purification in a conventional manner where, for example, the solvent and the unreacted monomer are to be distilled away under a reduced pressure.

As the reaction solvent, there may be used an aprotic organic solvent. For example, there may be listed ethyl acetate, propionitrile, toluene, xylene, bromobenzene, dimethoxyethane, diethoxyethane, diethyl ether, tetramethylene sulfone, N,N-dimethylformamide, N,N-dimethylacetamide, N-methylpyrrolidone, anisole, 2-butoxyethoxytrimethylsilane, cellosolve acetate, crown ether, acetonitrile and tetrahydrofuran (THF). In terms of reaction efficiency, preferred are dichloromethane, toluene, acetonitrile and tetrahydrofuran, of which tetrahydrofuran is more preferred.

A reaction temperature for the group transfer polymerization reaction is -100 to 150°C, preferably 0 to 50°C, more preferably 10 to 30°C.

A temperature at the time of distilling away the solvent and unreacted monomer under a reduced pressure is 80 to 300°C, preferably 100 to 200°C, more preferably 120 to 180°C. Further, a pressure at the time of performing stripping is not higher than 1 atm, preferably not higher than 0.1 atm, more preferably not higher than 0.007 atm.

As the catalyst, there may be generally used one selected from an anionic catalyst, a Lewis acid catalyst and an organic molecular catalyst that are known as catalysts for group transfer polymerization.

Examples of the anionic catalyst include tris(dimethylamino)sulfonium difluorotrimethylsilicate, tris(dimethylamino)sulfonium cyanide, tetraphenylarsonium cyanide, tris(dimethylamino)sulfonium azide, tetraethylammonium azide, bis(dialkylaluminum)oxide, borontrifluoride etherate, alkali metal fluoride, alkali metal cyanide, alkali metal azide, tris(dimethylamino)sulfonium difluorotriphenylstanate, tetrabutylammonium fluoride, tetramethylammonium fluoride, tetraethylammonium cyanide, tetrabutylammonium benzoate, tetrabutylammonium bibenzoate, and tetrabutylammonium m-chlorobenzoate.

Examples of the Lewis acid catalyst include zinc iodide, zinc bromide, zinc chloride, mono and dialkylaluminum halides, and dialkylaluminum oxide.

Examples of the organic molecular catalyst include 1,3-diisopropyl-4,5-dimethylimidazol-2-ylidene, 1,3-diisopropylimidazol-2-ylidene, 1,3-di-tert-butylimidazol-2-ylidene, 1,8-diazabicyclo[5.4.0]-7-undecene, 2,8,9-trimethyl-2,5,8,9-tetraaza-1-phosphabicyclo[3.3.3]undecane, 2,8,9-triisobutyl-2,5,8,9-tetraaza-1-phosphabicyclo[3.3.3]undecane, 1-tert-butyl-2,2,4,4,4-pentakis(dimethylamino)-2λ⁵,4λ⁵-catenadi(phosphazene), 1-tert-butyl-4,4,4-tris(dimethylamino)-2,2-bis[tris(dimethylamino)-phosphoranilidenamino]-2λ⁵,4λ⁵-catenadi(phosphazene), tris(2,4,6-trimethoxyphenyl)phosphine, tris-(pentafluorophenyl)borane, triethylsilyl trifluoromethanesulfonate, triphenylcarbenium tetrakis(pentafluorophenyl)borate, trifluoromethanesulfonimide, and 1-[bis(trifluoromethanesulfonyl)methyl]-2,3,4,5,6-pentafluorobenzene.

As the reaction terminator, a compound capable of donating protons is used. Examples thereof include methanol, isopropyl alcohol, n-butyl alcohol and water.

### [Emulsion composition]

An emulsion composition employed in the present invention contains the above diblock copolymer as an emulsifier, a water phase component, and an oil phase component. There are contained 0.1 to 10% by mass of the emulsifier, 5 to 90% by mass of the water phase component, and 5 to 60% by mass of the oil phase component, with respect to the total mass of the emulsion composition; in a wide range of emulsification compositions, the emulsion composition employed in the present invention is an emulsion composition superior in dispersion stability over time, and it is particularly preferred that the emulsion composition be a water-in-oil type emulsion composition.

As the water phase component and oil phase component that are to be contained in the emulsion composition, they can be appropriately selected depending on the use and purpose of the composition, examples of which may include later-described components known as cosmetic material components.

The water phase component contained in the emulsion composition contains water as its main component, and further contains various water-soluble components.

Examples of the oil phase component contained in the emulsion composition include a silicone oil, a hydrocarbon oil, a higher fatty acid, a polar oil such as an ester oil and a natural animal or vegetable oil, a semisynthetic oil, and/or a fluorine-based oil, of which a polar oil and a silicone oil are preferred.

Utilizing its emulsifying capability, the diblock copolymer employed in the present invention as an emulsifier can be used for various purposes; particularly, the diblock copolymer is preferable as a cosmetic raw material, and can be added to, for example, a basic cosmetic material such as a milky lotion, a cream, a beauty lotion, a facial pack, a dispersion liquid and a cleansing material; a makeup cosmetic material such as a foundation, a face powder, a lipstick, a blusher, an eyeshadow, an eyeliner and a mascara; and a hair cosmetic material such as a shampoo, a hair conditioner, a hair treatment agent and a hair styling material. In such case, it is preferred that the diblock copolymer of the present invention as an emulsifier be added to the cosmetic material by an amount of 0.01 to 10% by mass. An amount of less than 0.01% by mass shall make it difficult for a satisfactory emulsifying capability to be exerted; further, an amount of greater than 10% by mass is not preferable because a poor feeling of use will be incurred as extensibility dulls due to an increased viscosity.

Further, if using the diblock copolymer employed in the present invention as an emulsifier in a cosmetic material, there are no particular restrictions on other cosmetic material components; there may be added cosmetic material components that are normally employed depending on the type of a product or a certain cosmetic purpose. Examples of such cosmetic material components include an oil agent raw material such as a fat and oil, a wax, a hydrocarbon, a silicone oil, a fatty acid, an alcohol, an ester and a lanolin; a powder raw material such as a white pigment, a coloring pigment, an extender pigment, a photoluminescent pigment, an organic powder and a hydrophobized pigment; a metallic soap; a surfactant; a multivalent alcohol; a polymer compound; water; an antioxidant; an ultraviolet absorber; a preservative; a tar pigment; a natural pigment; a beauty component; and a perfume. These cosmetic material components may be appropriately added on the premise that the effects of the present invention will not be impaired.

An emulsion composition obtained using the diblock copolymer employed in the present invention as an emulsifier and a cosmetic material containing such emulsion composition are also part of the disclosure but are not claimed.

### WORKING EXAMPLES

The present invention is described in greater detail hereunder with reference to working examples; however, the present invention shall not be limited to these working examples. Here, in the examples below, unless otherwise noted, "%" in composition refers to % by mass.

### [Synthesis example 1]

Here, 19.9 mg of a tetrabutylammonium m-chlorobenzoate that had been dried under a reduced pressure was dissolved in 25 mL of THF. Under a nitrogen atmosphere, 436.0 mg of 1-methoxy-1-(trimethylsiloxy)-2-methyl-1-propene as an initiator was added to such THF solution of tetrabutylammonium m-chlorobenzoate, followed by spending 30 min delivering 7.5 g of the following silicone macromer (a) thereinto by drops so as to prepare a reaction solution. This reaction solution was further stirred at room temperature for another two hours. Next, 5 min were spent delivering 7.5 g of 2-(dimethylamino)ethylmethacrylate by drops into this reaction solution, and the solution was then stirred at room temperature for an hour, followed by adding 10 mL of methanol thereinto so as to terminate the reaction. The reaction solution after the termination of the reaction was then subjected to stripping at 105°C and a reduced pressure of lower than 0.007 atm for an hour, thereby obtaining a target diblock copolymer.

Here, gel permeation chromatography (GPC) analysis was conducted at two time points which were the time point at which the group transfer polymerization of the silicone macromer (a) had finished; and a time point after the reaction was over. As a result, at each stage, there was confirmed an increase in molecular weight according to the preparation ratio between the initiator and monomer, and it was confirmed that the target diblock copolymer had been obtained.

The number average molecular weight of the diblock copolymer of the synthesis example 1, the polydispersity of the molecular weight thereof, and a polymerization degree ratio between each monomer were as follows.

Number average molecular weight (Mn)=5025, Polydispersity (Mw/Mn)=1.19, Polymerization degree ratio (n²/n¹)=5.34

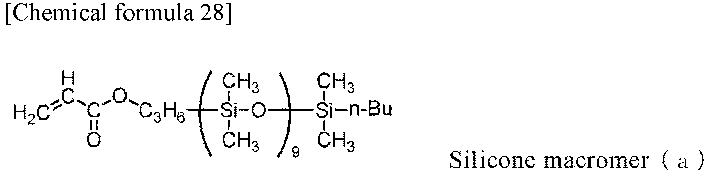

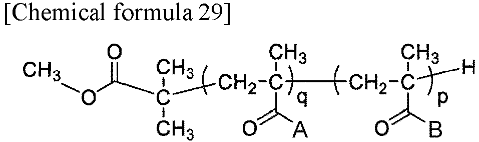

(A represents a residue of the silicone macromer (a), and B represents a residue of 2-(dimethylamino)ethylmethacrylate. The average polymerization degree q of the silicone macromer (a) is q=3.5, and the average polymerization degree p of 2-(dimethylamino)ethylmethacrylate is p=18.4.)

### [Synthesis example 2]

Here, 19.9 mg of a tetrabutylammonium m-chlorobenzoate that had been dried under a reduced pressure was dissolved in 25 mL of THF. Under a nitrogen atmosphere, 436.0 mg of 1-methoxy-1-(trimethylsiloxy)-2-methyl-1-propene as an initiator was added to such THF solution of tetrabutylammonium m-chlorobenzoate, followed by spending 30 min delivering 11.76 g of the silicone macromer (a) thereinto by drops so as to prepare a reaction solution. This reaction solution was further stirred at room temperature for another hour. Next, 15 min were spent delivering 3.24 g of the following monomer (b) by drops into this reaction solution, and the solution was then stirred at room temperature for three hours, followed by adding 10 mL of methanol thereinto so as to terminate the reaction. The reaction solution after the termination of the reaction was then subjected to stripping at 105°C and a reduced pressure of lower than 0.007 atm for an hour, thereby obtaining a target diblock copolymer.

In a similar manner as the synthesis example 1, it was confirmed that the product obtained was a diblock copolymer. The number average molecular weight of the diblock copolymer of the synthesis example 2, the polydispersity of the molecular weight thereof, and a polymerization degree ratio between each monomer were as follows.

Number average molecular weight (Mn)=9318, Polydispersity (Mw/Mn)=1.21, Polymerization degree ratio (n²/n¹)=1.00

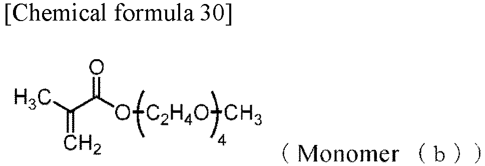

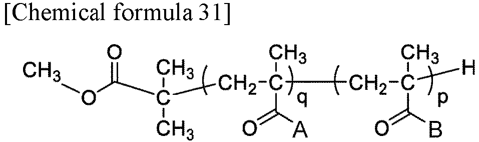

(A represents a residue of the silicone macromer (a), and B represents a residue of the monomer (b). The average polymerization degree q of the silicone macromer (a) is q=7.9, and the average polymerization degree p of the monomer (b) is p=7.9.)

### [Synthesis example 3]

Here, 19.9 mg of a tetrabutylammonium m-chlorobenzoate that had been dried under a reduced pressure was dissolved in 25 mL of THF. Under a nitrogen atmosphere, 436.0 mg of 1-methoxy-1-(trimethylsiloxy)-2-methyl-1-propene as an initiator was added to such THF solution of tetrabutylammonium m-chlorobenzoate, followed by spending an hour delivering 9.67 g of the silicone macromer (a) thereinto by drops so as to prepare a reaction solution. This reaction solution was further stirred at room temperature for another two hours. Next, 30 min were spent delivering 5.33 g of the following monomer (c) by drops into this reaction solution, and the solution was then stirred at room temperature for five hours, followed by adding 10 mL of methanol thereinto so as to terminate the reaction. The reaction solution after the termination of the reaction was then subjected to stripping at 105°C and a reduced pressure of lower than 0.007 atm for an hour, thereby obtaining a target diblock copolymer.

In a similar manner as the synthesis example 1, it was confirmed that the product obtained was a diblock copolymer. The number average molecular weight of the diblock copolymer of the synthesis example 3 and the polydispersity of the molecular weight thereof were as follows.

Number average molecular weight (Mn)=9327, Polydispersity (Mw/Mn)=1.24, Polymerization degree ratio (n²/n¹)=0.73

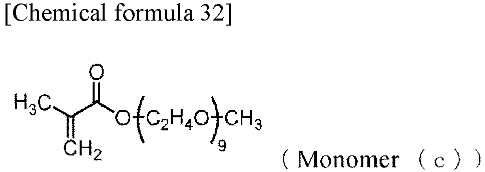

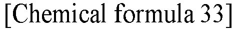

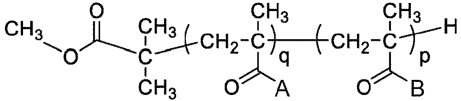

(A represents a residue of the silicone macromer (a), and B represents a residue of the monomer (c). The average polymerization degree q of the silicone macromer (a) is q=7.4, and the average polymerization degree p of the monomer (c) is p=5.4.)

### [Comparative synthesis example 1]

Here, 19.9 mg of a tetrabutylammonium m-chlorobenzoate that had been dried under a reduced pressure was dissolved in 25 mL of THF. Under a nitrogen atmosphere, 436.0 mg of 1-methoxy-1-(trimethylsiloxy)-2-methyl-1-propene as an initiator was added to such THF solution of tetrabutylammonium m-chlorobenzoate, followed by spending 30 min delivering 7.5 g of the silicone macromer (a) and 7.5 g of 2-(dimethylamino)ethylmethacrylate thereinto by drops so as to prepare a reaction solution. This reaction solution was then stirred at room temperature for an hour, followed by adding 10 mL of methanol thereinto so as to terminate the reaction. The reaction solution after the termination of the reaction was then subjected to stripping at 105°C and a reduced pressure of lower than 0.007 atm for an hour, thereby obtaining a target random copolymer. The number average molecular weight of the random copolymer of the comparative synthesis example 1 and the polydispersity of the molecular weight thereof were as follows.

Number average molecular weight (Mn)=5302, Polydispersity (Mw/Mn)=1.32

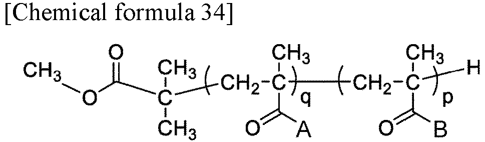

(A represents a residue of the silicone macromer (a), and B represents a residue of 2-(dimethylamino)ethylmethacrylate. The average polymerization degree q of the silicone macromer (a) is q=2.9, and the average polymerization degree p of 2-(dimethylamino)ethylmethacrylate is p=16.9.)

### [Stability evaluation of emulsion composition]

An emulsion composition was produced in accordance with the compositions shown in Table 1; the preservation stability of the emulsion composition obtained was then evaluated under the following criteria based on, for example, the presence or non-presence of phase separation and a gel-like substance after being stored at 40°C for three days.
∘: Phase separation was not observed at all, and a gel-like substance was not observed either.
×: Liquid was separated, or a gel-like substance was generated.

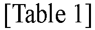

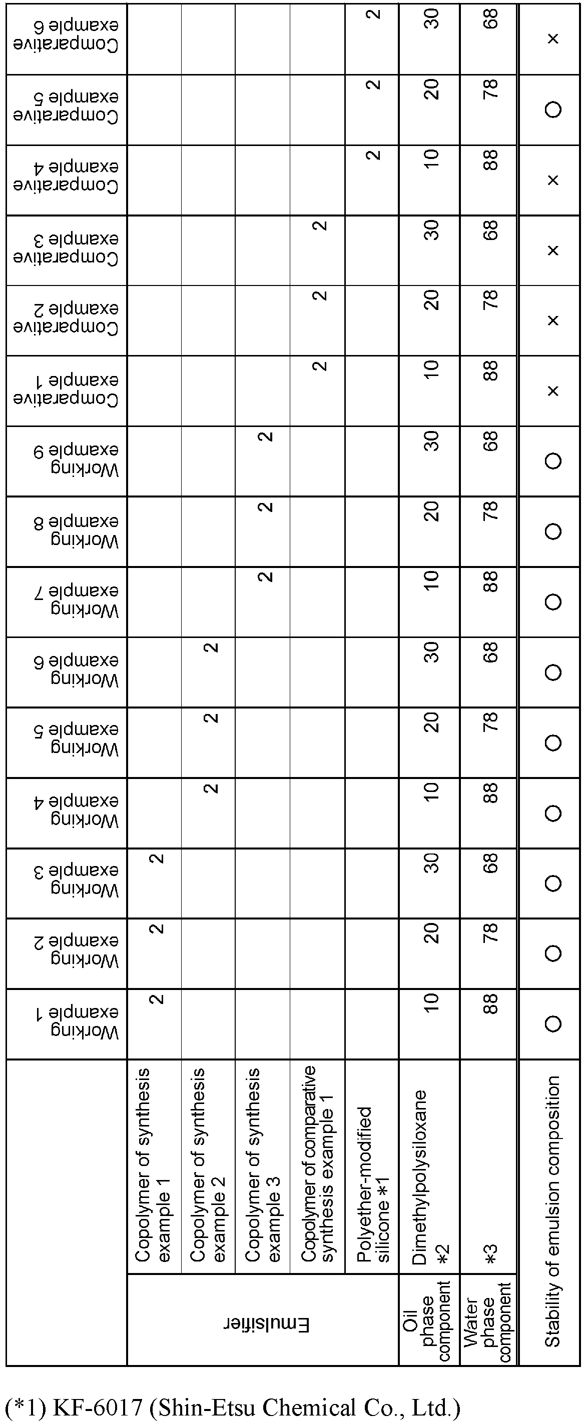

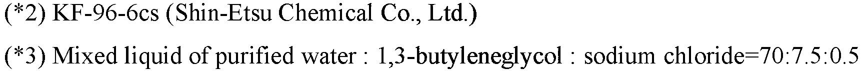

As is clear from the results shown in Table 1, when compared to the random copolymer (comparative synthesis example 1) synthesized using the same monomer, the diblock copolymer employed in the present invention has an excellent emulsifying capability and is capable of providing an emulsion composition superior in stability. Further, it became clear that there can be provided a stable emulsion composition with a wider range of allowable water content as compared to a polyether-modified silicone.

## Claims

1. Use of a diblock copolymer as an emulsifier, wherein a main chain of the diblock copolymer is comprised of a silicone graft copolymer block represented by a formula [I] and a polar copolymer block represented by a formula [II], wherein one end structure of the main chain is represented by a formula [III], and the other end structure of the main chain is represented by a formula [IV], the formula [I] being expressed as 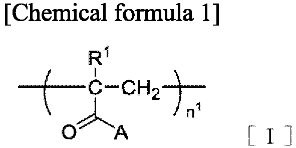
wherein R¹ represents a hydrogen atom or a methyl group,
A represents an organopolysiloxane-containing group represented by a general formula (1) or an organopolysiloxane-containing group represented by a general formula (2),
n¹ represents a number of repeating units and satisfies 1≤ n¹ ≤50,
wherein the general formula (1) is expressed as 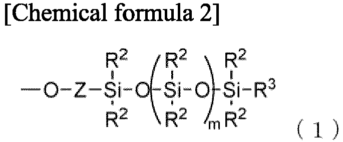
wherein the organopolysiloxane-containing group represented by the general formula (1) has a linear organopolysiloxane structure where a repeating unit number m is 0 to 100,
Z represents a divalent organic group,
each R² independently represents a saturated hydrocarbon group having 1 to 10 carbon atoms or a phenyl group,
R³ represents a saturated hydrocarbon group having 1 to 10 carbon atoms,
m is a number of 0 to 100, and
wherein the general formula (2) is expressed as
[Chemical formula 3]
-O-Lⁱ (2)
wherein the organopolysiloxane-containing group represented by the general formula (2) has a dendritic organopolysiloxane structure whose hierarchical number c is 1 to 10,
Lⁱ is a silylorgano group represented by a general formula (3),
i represents a number of each hierarchy of the dendritic structure and is each integer from 1 to c in both the general formulae (2) and (3), and is 1 in the general formula (2),
wherein the general formula (3) is expressed as 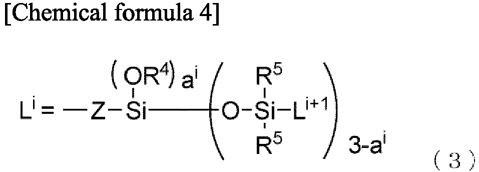
wherein Z represents a divalent organic group,
R⁴ represents a saturated hydrocarbon group having 1 to 10 carbon atoms or a phenyl group,
each R⁵ independently represents an alkyl group having 1 to 8 carbon atoms or a phenyl group,
Lⁱ⁺¹ is the silylorgano group Lⁱ represented by the general formula (3) when the hierarchy i is lower than c (lower than the uppermost hierarchy); and is a hydrogen atom, a saturated hydrocarbon group having 1 to 10 carbon atoms or a phenyl group, when the hierarchy i is i=c (the uppermost hierarchy),
aⁱ represents the number of OR⁴ groups in the hierarchy i and is a number of 0 to 3;
the formula [II] being expressed as 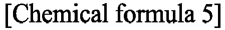 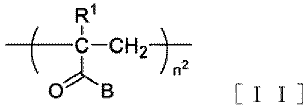
wherein R¹ represents a hydrogen atom or a methyl group,
B is any group represented by a general formula (8') or (9')
[Chemical formulae 16] -O-CH₂-CH₂-N(R⁷)₂ (8' ) -O-(C₂H₄O)_{n³}-R³ (9' )
wherein in the formula (8'), each R⁷ independently represents a hydrogen atom or an alkyl group having 1 to 4 carbon atoms; in the formula (9'), R³ represents a saturated hydrocarbon group having 1 to 10 carbon atoms; and n³ represents a number of the repeating units, provided that 1≤n³≤10,
n² represents a number of repeating units and satisfies 1≤ n²≤50; the formula [III] being expressed as 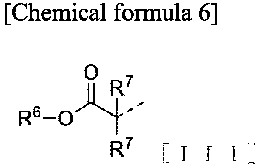
wherein R⁶ represents an alkyl group having 1 to 4 carbon atoms,
each R⁷ independently represents a hydrogen atom or an alkyl group having 1 to 4 carbon atoms; the formula [IV] being expressed as 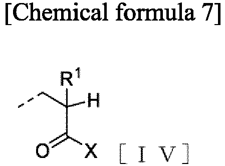
wherein R¹ represents a hydrogen atom or a methyl group, X represents the group represented by A in the formula [I] or the group represented by B in the formula [II].

2. The use according to claim 1, wherein in the formula [II], B is a group represented by the general formula (8').

3. The use according to claim 1 or 2, wherein the diblock copolymer has a number average molecular weight of 2,000 to 25,000 in terms of polystyrene when measured by gel permeation chromatography.

4. The use according to any of the claims 1 to 3, wherein the the diblock copolymer is contained in an emulsion composition, the emulsion composition comprising the deblock copolymer a water phase component and an oil phase component.

5. The use according to claim 4, wherein in the emulsion composition
the emulsifier is in an amount of 0.1 to 10% by mass,
the water phase component is in an amount of 5 to 90% by mass, and
the oil phase component is in an amount of 5 to 60% by mass, with respect to a total mass of the emulsion composition.

6. The use according to claim 5, wherein the emulsion composition is used as a cosmetic material.

## Patentansprüche

1. Verwendung eines Diblockcopolymers als Emulgator, wobei eine Hauptkette des Diblockcopolymers aus einem Silikonpfropfcopolymerblock, der durch eine Formel [I] dargestellt ist, und einem polaren Copolymerblock, der durch eine Formel [II] dargestellt ist, besteht, wobei eine Endstruktur der Hauptkette durch eine Formel [III] dargestellt ist und die andere Endstruktur der Hauptkette durch eine Formel [IV] dargestellt ist, wobei die Formel [I] ausgedrückt ist als 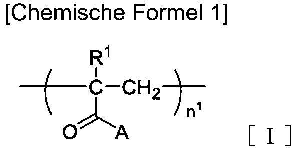
wobei R¹ ein Wasserstoffatom oder eine Methylgruppe darstellt,
A eine Organopolysiloxan enthaltende Gruppe, die durch eine allgemeine Formel (1) dargestellt ist, oder eine Organopolysiloxan enthaltende Gruppe, die durch eine allgemeine Formel (2) dargestellt ist, darstellt,
n¹ eine Anzahl von Wiederholungseinheiten darstellt und 1 ≤ n¹ ≤ 50 erfüllt,
wobei die allgemeine Formel (1) ausgedrückt ist als 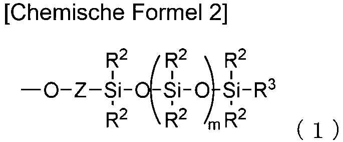
wobei die Organopolysiloxan enthaltende Gruppe, die durch die allgemeine Formel (1) dargestellt ist, eine lineare Organopolysiloxanstruktur aufweist, wobei eine Wiederholungseinheitszahl m 0 bis 100 beträgt,
Z eine zweiwertige organische Gruppe darstellt,
jedes R² unabhängig eine gesättigte Kohlenwasserstoffgruppe mit 1 bis 10 Kohlenstoffatomen oder eine Phenylgruppe darstellt,
R³ eine gesättigte Kohlenwasserstoffgruppe mit 1 bis 10 Kohlenstoffatomen darstellt, m eine Zahl von 0 bis 100 ist, und
wobei die allgemeine Formel (2) ausgedrückt ist als
[Chemische Formel 3]
-O-Lⁱ (2)
wobei die Organopolysiloxan enthaltende Gruppe, die durch die allgemeine Formel (2) dargestellt ist, eine dendritische Organopolysiloxanstruktur aufweist, deren hierarchische Zahl c 1 bis 10 beträgt,
Lⁱ eine Silylorganogruppe ist, die durch eine allgemeine Formel (3) dargestellt ist,
i eine Zahl jeder Hierarchie der dendritischen Struktur darstellt und jede ganze Zahl von 1 bis c in beiden allgemeinen Formeln (2) und (3) ist und 1 in der allgemeinen Formel (2) ist,
wobei die allgemeine Formel (3) ausgedrückt ist als 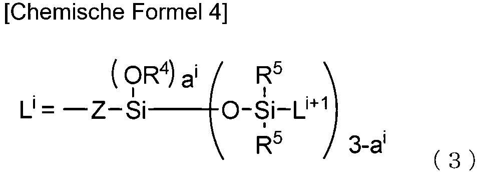
wobei Z eine zweiwertige organische Gruppe darstellt,
R⁴ eine gesättigte Kohlenwasserstoffgruppe mit 1 bis 10 Kohlenstoffatomen oder eine Phenylgruppe darstellt,
jedes R⁵ unabhängig eine Alkylgruppe mit 1 bis 8 Kohlenstoffatomen oder eine Phenylgruppe darstellt,
Lⁱ⁺¹ die Silylorganogruppe Lⁱ ist, die durch die allgemeine Formel (3) dargestellt ist, wenn die Hierarchie i niedriger als c (niedriger als die oberste Hierarchie) ist; und ein Wasserstoffatom, eine gesättigte Kohlenwasserstoffgruppe mit 1 bis 10 Kohlenstoffatomen oder eine Phenylgruppe ist, wenn die Hierarchie i i=c (die oberste Hierarchie) ist,
aⁱ die Zahl von OR⁴ -Gruppen in der Hierarchie i darstellt und eine Zahl von 0 bis 3 ist; wobei die Formel [II] ausgedrückt ist als 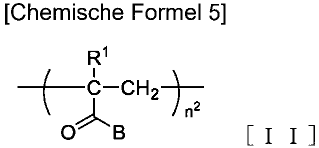
wobei R¹ ein Wasserstoffatom oder eine Methylgruppe darstellt,
B eine beliebige Gruppe ist, die durch eine allgemeine Formel (8') oder (9') dargestellt ist
[Chemische Formeln 16] -O-CH₂-CH₂-N(R⁷)₂ (8' ) -O-(C₂H₄O)_{n³}-R³ (9' )
wobei in der Formel (8') jedes R⁷ unabhängig ein Wasserstoffatom oder eine Alkylgruppe mit 1 bis 4 Kohlenstoffatomen darstellt; in der Formel (9') R³ eine gesättigte Kohlenwasserstoffgruppe mit 1 bis 10 Kohlenstoffatomen darstellt; und n³ eine Anzahl der Wiederholungseinheiten darstellt, vorausgesetzt, dass 1 ≤ n³ ≤ 10,
n² eine Anzahl von Wiederholungseinheiten darstellt und 1 ≤ n² ≤ 50 erfüllt; wobei die Formel [III] ausgedrückt ist als 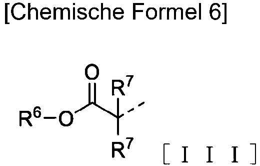
wobei R⁶ eine Alkylgruppe mit 1 bis 4 Kohlenstoffatomen darstellt,
jedes R⁷ unabhängig ein Wasserstoffatom oder eine Alkylgruppe mit 1 bis 4 Kohlenstoffatomen darstellt; wobei die Formel [IV] ausgedrückt ist als 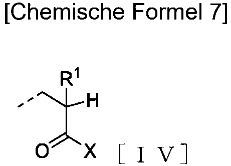
wobei R¹ ein Wasserstoffatom oder eine Methylgruppe darstellt,
X die Gruppe, die durch A in der Formel [I] dargestellt ist, oder die Gruppe, die durch B in der Formel [II] dargestellt ist, darstellt.

2. Verwendung nach Anspruch 1, wobei in der Formel [II] B eine Gruppe ist, die durch die allgemeine Formel (8') dargestellt ist.

3. Verwendung nach Anspruch 1 oder 2, wobei das Diblockcopolymer ein Zahlenmittel des Molekulargewichts von 2.000 bis 25.000 in Bezug auf Polystyrol aufweist, wenn es durch Gelpermeationschromatographie gemessen wird.

4. Verwendung nach einem der Ansprüche 1 bis 3, wobei das Diblockcopolymer in einer Emulsionszusammensetzung enthalten ist, wobei die Emulsionszusammensetzung das Deblockcopolymer, eine Wasserphasenkomponente und eine Ölphasenkomponente umfasst.

5. Verwendung nach Anspruch 4, wobei in der Emulsionszusammensetzung
der Emulgator in einer Menge von 0,1 bis 10 Massen-% vorliegt,
die Wasserphasenkomponente in einer Menge von 5 bis 90 Massen-% vorliegt, und
die Ölphasenkomponente in einer Menge von 5 bis 60 Massen-% vorliegt, bezogen auf eine Gesamtmasse der Emulsionszusammensetzung.

6. Verwendung nach Anspruch 5, wobei die Emulsionszusammensetzung als ein kosmetisches Material verwendet wird.

## Revendications

1. Utilisation d'un copolymère dibloc en tant qu'émulsifiant, dans laquelle une chaîne principale du copolymère dibloc est constituée d'un bloc de copolymère greffé de silicone représenté par la formule [I] et d'un bloc de copolymère polaire représenté par la formule [II], dans laquelle une structure d'extrémité de la chaîne principale est représentée par la formule [III] et l'autre structure d'extrémité de la chaîne principale est représentée par la formule [IV], la formule [I] étant exprimée comme suit 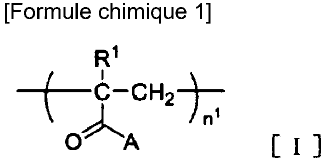
dans laquelle R¹ représente un atome d'hydrogène ou un groupe méthyle,
A représente un groupe contenant un organopolysiloxane représenté par la formule générale (1) ou un groupe contenant un organopolysiloxane représenté par la formule générale (2),
n¹ représente un nombre de motifs répétitifs et satisfait 1 ≤ n¹ ≤ 50,
dans laquelle la formule générale (1) est exprimée comme suit 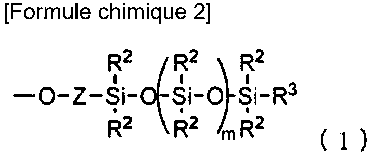
dans laquelle le groupe contenant un organopolysiloxane représenté par la formule générale (1) possède une structure organopolysiloxane linéaire dans laquelle un nombre de motifs répétitifs m est de 0 à 100,
Z représente un groupe organique divalent,
chaque R² représente indépendamment un groupe hydrocarboné saturé ayant de 1 à 10 atomes de carbone ou un groupe phényle,
R³ représente un groupe hydrocarboné saturé ayant de 1 à 10 atomes de carbone,
m est un nombre de 0 à 100, et
dans laquelle la formule générale (2) est exprimée comme suit
[Formule chimique 3]
-O-Lⁱ (2)
dans laquelle le groupe contenant un organopolysiloxane représenté par la formule générale (2) possède une structure organopolysiloxane dendritique dont le nombre hiérarchique c est de 1 à 10,
Lⁱ est un groupe silylorgano représenté par la formule générale (3),
i représente un nombre de chaque hiérarchie de la structure dendritique et est chacun un entier de 1 à c dans les deux formules générales (2) et (3), et vaut 1 dans la formule générale (2),
dans laquelle la formule générale (3) est exprimée comme suit 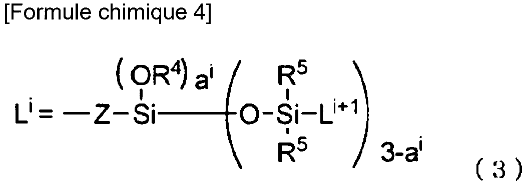
dans laquelle Z représente un groupe organique divalent,
R⁴ représente un groupe hydrocarboné saturé ayant de 1 à 10 atomes de carbone ou un groupe phényle,
chaque R⁵ représente indépendamment un groupe alkyle ayant de 1 à 8 atomes de carbone ou un groupe phényle,
Lⁱ⁺¹ est le groupe silylorgano Lⁱ représenté par la formule générale (3) lorsque la hiérarchie i est inférieure à c (inférieure à la hiérarchie supérieure) ; et est un atome d'hydrogène, un groupe hydrocarboné saturé ayant de 1 à 10 atomes de carbone ou un groupe phényle, lorsque la hiérarchie i est i= c (la hiérarchie supérieure),
aⁱ représente le nombre de groupes OR⁴ dans la hiérarchie i et vaut un nombre de 0 à 3 ;
la formule (III) étant exprimée comme suit 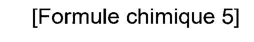 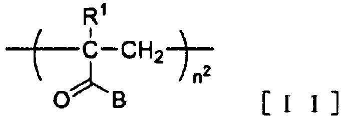
dans laquelle R¹ représente un atome d'hydrogène ou un groupe méthyle,
B est un groupe quelconque représenté par la formule générale (8') ou (9')
[Formules chimiques 16] -O-CH₂-CH₂-N(R⁷)₂ (8' ) -O-(C₂H₄O)_{n³}-R³ (9' )
dans laquelle dans la formule (8'), chaque R⁷ représente indépendamment un atome d'hydrogène ou un groupe alkyle ayant 1 à 4 atomes de carbone ; dans la formule (9'), R³ représente un groupe hydrocarboné saturé ayant de 1 à 10 atomes de carbone ; et n³ représente un nombre de motifs répétitifs, à condition que 1 ≤ n³ ≤ 10,
n² représente un nombre de motifs répétitifs et satisfait à 1 ≤ n² ≤ 50 ; la formule [III] étant exprimée comme suit 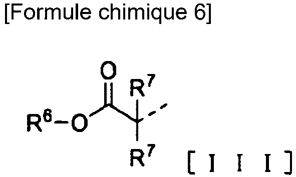
dans laquelle R⁶ représente groupe alkyle ayant 1 à 4 atomes de carbone,
chaque R⁷ représente indépendamment un atome d'hydrogène ou un groupe alkyle ayant 1 à 4 atomes de carbone ; la formule [IV] étant exprimée comme suit 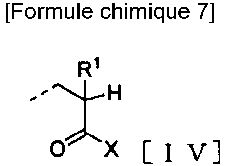
dans laquelle R¹ représente un atome d'hydrogène ou un groupe méthyle,
X représente le groupe A dans la formule [I] ou le groupe B dans la formule [II].

2. Utilisation selon la revendication 1, dans laquelle dans la formule [II], B est un groupe représenté par la formule générale (8').

3. Utilisation selon la revendication 1 ou 2, dans laquelle le copolymère dibloc a un poids moléculaire moyen en nombre de 2 000 à 25 000 en termes de polystyrène lorsqu'elle est mesurée par chromatographie par perméation de gel.

4. Utilisation selon l'une quelconque des revendications 1 à 3, dans lequel le copolymère dibloc est contenu dans une composition d'émulsion, la composition d'émulsion comprenant le copolymère dibloc, un composant de phase aqueuse et un composant de phase huileuse.

5. Utilisation selon la revendication 4, dans laquelle dans la composition d'émulsion
l'émulsifiant est présent en une quantité de 0,1 à 10 % en masse,
le composant de phase aqueuse est présent en une quantité de 5 à 90 % en masse, et
le composant de phase huileuse est présent en une quantité de 5 à 60 % en masse, par rapport à la masse totale de la composition d'émulsion.

6. Utilisation selon la revendication 5, dans laquelle dans la composition d'émulsion est utilisée comme substance cosmétique.
